# EUROPÄISCHE PATENTSCHRIFT

(11) **EP 1 785 730 B1**
(45) Veröffentlichungstag und Bekanntmachung des Hinweises auf die Patenterteilung: **13.02.2013**
(21) Anmeldenummer: 05024897.0
(22) Anmeldetag: 15.11.2005
(51) Int. Cl.: A61B 5/145, G01N 21/84, G01N 35/00

(54) **System und Verfahren zur Untersuchung einer Probenflüssigkeit**
System and method for examining a liquid sample
Système et procédé destiné à I'analyse d'un échantillon liquide

(43) Veröffentlichungstag der Anmeldung: 16.05.2007
(73) Patentinhaber: F.Hoffmann-La Roche AG, 4070 Basel (CH); Roche Diagnostics GmbH, 68305 Mannheim (DE)
(72) Erfinder: Krämer, Uwe, Dr., 68549 Ilvesheim (DE); List, Hans, 64754 Hesseneck-Kailbach (DE)
(74) Vertreter: Pfiz, Thomas

(56) Entgegenhaltungen:
- WO-A-2004/056269
- DE-A1- 19 819 407
- US-A- 4 867 946
- US-A1- 2002 192 833
- US-B1- 6 707 554

## Beschreibung

Die Erfindung betrifft ein System zur Untersuchung einer Probenflüssigkeit, insbesondere für Blutglucosebestimmungen, mit einem eine Mehrzahl von Testelementen aufweisenden, vorzugsweise in einer Bandkassette gelagerten Testband, einer die Testelemente unter Bandvorlauf sukzessive an eine Probenaufgabestelle transportierenden Bandtransportvorrichtung und einer die mit Probenflüssigkeit beaufschlagten Testelemente an einem Messort abtastenden Messeinrichtung, wobei der Messort in Bandlaufrichtung im Abstand von der Probenaufgabestelle angeordnet ist. Die Erfindung betrifft auch ein korrespondierendes Verfahren.

Die WO 2004/056269 beschreibt ein Testsystem für Körperflüssigkeiten mit einem Testband zur Bereitstellung einer Vielzahl von auf einem Trägerband abschnittsweise aufgebrachten Testeinheiten in einem kompakten Handgerät. Damit sollen bei in der Regel täglich mehrfach durchgeführten Blutzucker-Selbstkontrollen der betroffenen Person möglichst wenige Handhabungsschritte auferlegt werden. Um eine möglichst geringe Blutmenge dosieren zu können, ist eine Umlenkung der Testelemente über eine Umlenkspitze vorgesehen, welche zugleich die Messstelle bildet. Daneben wird auch erwähnt, dass eine Messposition im Abstand von der Sammelposition es erlaubt, eine Ausleseoptik oder elektrochemische Analyseeinheit im Gerät gesondert zu positionieren.

Die US 6,707,554 B1 beschreibt einphotometrisches Analysesystem für Testelemente in Form von Teststreifen, welche über Geräteanschläge positioniert werden. Um hierbei Toleranzen ausgleichen zu können, sind mehrere Punktlichtquellen zur Beleuchtung unterschiedlicher Bereiche der Detektionszone auf dem Teststreifen vorgesehen, wobei dann eine Auswahl anhand der erhaltenen Signale getroffen werden soll.

Ausgehend hiervon liegt der Erfindung die Aufgabe zugrunde, die im Stand der Technik bekannten Systeme weiter zu verbessern, insbesondere die Möglichkeit für einen optimierten Geräteaufbau zu schaffen und mit möglichst geringen Probenmengen eine zuverlässige Messung zu ermöglichen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine gezielte Vermessung der Testelemente ohne mechanische Positionierelemente zu ermöglichen. Dementsprechend wird erfindungsgemäß eine auf das Vorhandensein von Probenflüssigkeit auf dem jeweils aktiven Testelement ansprechende, die Bandtransportvorrichtung ansteuernde Positioniereinrichtung vorgeschlagen, um den Bandtransport zur Positionierung eines Probenflüssigkeit tragenden Benetzungsbereichs der Testelemente am Messort zu unterbrechen. Auf diese Weise ist es möglich, eine größere Testfläche für den Probenauftrag bereit zu stellen, ohne dass eine vollflächige Bedeckung erforderlich wäre. Speziell bei Blutzuckermessungen genügen somit mikroskopische Blutmengen, welche sich schmerzarm gewinnen lassen, wobei ein zusätzlicher Komfort durch eine integrierte Stecheinheit geschaffen werden kann, weil der Sammelort nicht durch eine Messeinheit verstellt wird. Durch die Transportpositionierung wird die Systemkonstruktion vereinfacht und der Bauraum besser nutzbar, und es kann sichergestellt werden, dass auch bei variablem Probenauftrag immer eine zielgenaue Detektion erfolgt.

Vorteilhafterweise umfasst die Positioniereinrichtung eine mit einem Bandantrieb gekoppelte, speziell durch eine Programmroutine eines Mikroprozessors realisierte Steuereinheit für einen gezielten Bandstopp am Messort. Eine weitere Verbesserung wird dadurch erzielt, dass die Positioniereinrichtung mindestens eine beim Durchlauf des Benetzungsbereichs ansprechende optische Erfassungseinheit aufweist.

Um eine genaue Probenzentrierung sicherzustellen, ist es von Vorteil, wenn die Positioniereinrichtung zwei auf Banddurchlaufstellen vor und nach einer Nutzsignalerfassungszone der Messeinrichtung ausgerichtete Lichtquellen aufweist. Dies lässt sich besonders einfach dadurch realisieren, dass die Positioniereinrichtung mehrere über eine gemeinsame Sammeloptik in Bandlaufrichtung hintereinander auf dem Testband als Leuchtflecken abgebildete Leuchtdioden aufweist.

Vorteilhafterweise besitzt die Positioniereinrichtung mindestens einen Photosensor zur vorzugsweise reflektometrischen Abtastung der Testelemente. Hierbei ist es günstig, wenn der Photosensor in einem unbenetzten Testfeldbereich einen Leerwert und im Benetzungsbereich einen davon verschiedenen Zielwert erfasst.

Um hohen Anforderungen an die Qualität der Signalerfassung zu genügen, ist es vorteilhaft, wenn die Messeinrichtung einen von der Positioniereinrichtung gesonderten Detektor für die Nutzsignalerfassung zum Nachweis eines Analyten in der Probenflüssigkeit besitzt.

Vorteilhafterweise sind die Testelemente durch vorzugsweise als Reagenzschicht ausgebildete flächige Testfelder auf dem Testband gebildet, wobei die Testfelder einen vorzugsweise durch eine Flüssigkeitsbarriere von der Probenflüssigkeit freigehaltenen Referenzbereich für eine Leerwertmessung aufweisen können.

Eine Feinpositionierung kann auch dadurch ermöglicht werden, dass das Testband mindestens abschnittsweise mit einer Bandmarkierung, insbesondere einem Strichmaßstab versehen ist, und dass die Positioniereinrichtung ein Abtastmittel, insbesondere eine Lichtschranke zum Abtasten der Bandmarkierung im Zuge des Bandvorlaufs aufweist.

Zur Reduzierung der erforderlichen Probenmenge ist es vorgesehen, dass der Benetzungsbereich der Testelemente einen Durchmesser von weniger als 5 mm, insbesondere von 1 bis 2 mm aufweist.

Um ausreichend Freiraum für die Messeinrichtung zu schaffen, ist es vorteilhaft, wenn der Messort in einem Abstand von 5 mm bis 5 cm von der Probenaufgabestelle liegt.

Eine weitere Verbesserung der Systemauslegung insbesondere für Blutzuckermessungen wird dadurch erreicht, dass an der Probenaufgabestelle eine Stecheinrichtung zum Einstechen in ein Körperteil vorzugsweise durch das Testband hindurch angeordnet ist.

In verfahrensmäßiger Hinsicht wird die eingangs genannte Aufgabe dadurch gelöst, dass durch eine auf das Vorhandensein von Probenflüssigkeit auf dem Testelement ansprechende Positioniereinrichtung der Bandtransport gesteuert wird, um einen Probenflüssigkeit tragenden Benetzungsbereich der Testelemente am Messort zu positionieren.

Im Folgenden wird die Erfindung anhand der in der Zeichnung schematisch dargestellten Ausführungsbeispiele näher erläutert. Es zeigen
- Fig. 1: ein Testbandsystem zur Blutglucosebestimmung in einer schematischen Darstellung;
- Fig. 2: eine Positioniereinrichtung zur Positionierung des Testbands am Messort als Blockschaltbild;
- Fig. 3: einen Ausschnitt eines mit Testelementen versehenen Testbands;
- Fig. 4: die auf das Testband ausgerichtete Positioniereinrichtung in zwei Ansichten;
- Fig. 5: verschiedene Benetzungen eines Testfelds mit zugeordneten Remissionssignalen; und
- Fig. 6: eine weitere Ausführungsform einer Positioniereinrichtung mit einem Testbandabschnitt in verschiedenen Vorlaufstellungen.

Das in der Zeichnung dargestellte Testsystem umfasst ein als Kassette in einem Handgerät 10 eingesetztes Testband 12 mit einer Vielzahl von darauf befindlichen Testelementen 14, eine Transportvorrichtung 16 für den Bandtransport, eine Messeinrichtung 18 zur optischen Untersuchung der Testelemente 14 und eine Positioniereinrichtung 20 zur Positionierung der Testfelder 14 im Erfassungsbereich der Messeinrichtung 18.

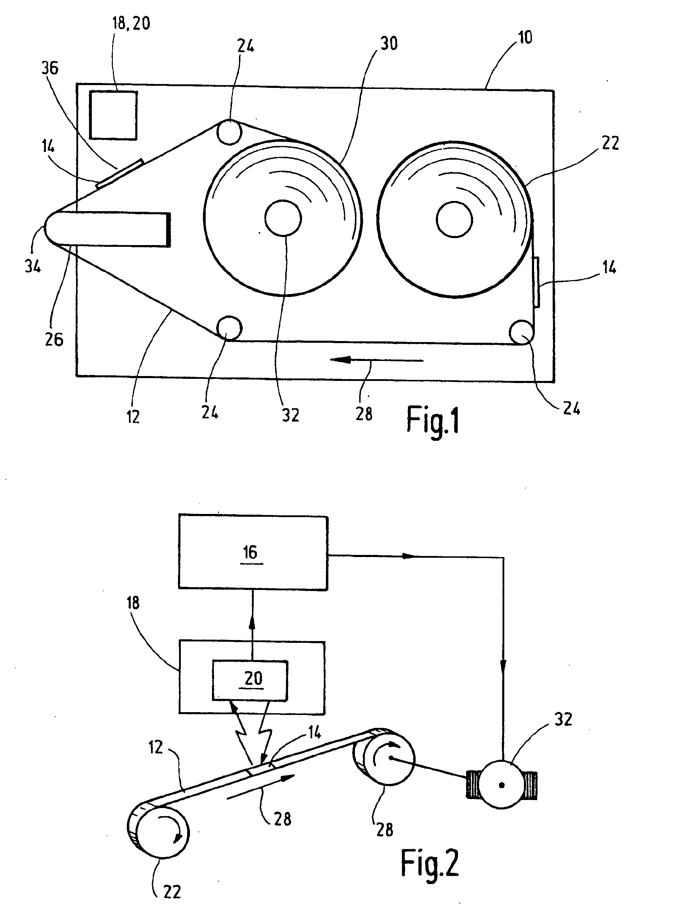

Wie in Fig. 1 und 2 veranschaulicht, kann das Testband 12 von einer Vorratsspule 22 abgezogen, über verschiedene Umlenkrollen 24 und eine Umlenkspitze 26 in Bandlaufrichtung (Pfeil 28) abgezogen und auf eine Aufwickelspule 30 wieder aufgewickelt werden. Der Bandvorlauf wird durch einen an der Aufwickelspule 30 angreifenden motorischen Bandantrieb 32 ermöglicht. Durch entsprechende Bandstopps können die Testelemente 14 im Bereich der Umlenkspitze 26 als Probenaufgabestelle gezielt mit Körperflüssigkeit (Blut) beaufschlagt und anschließend geräteintern am Messort 36 mittels der Messeinrichtung 18 optisch abgetastet werden.

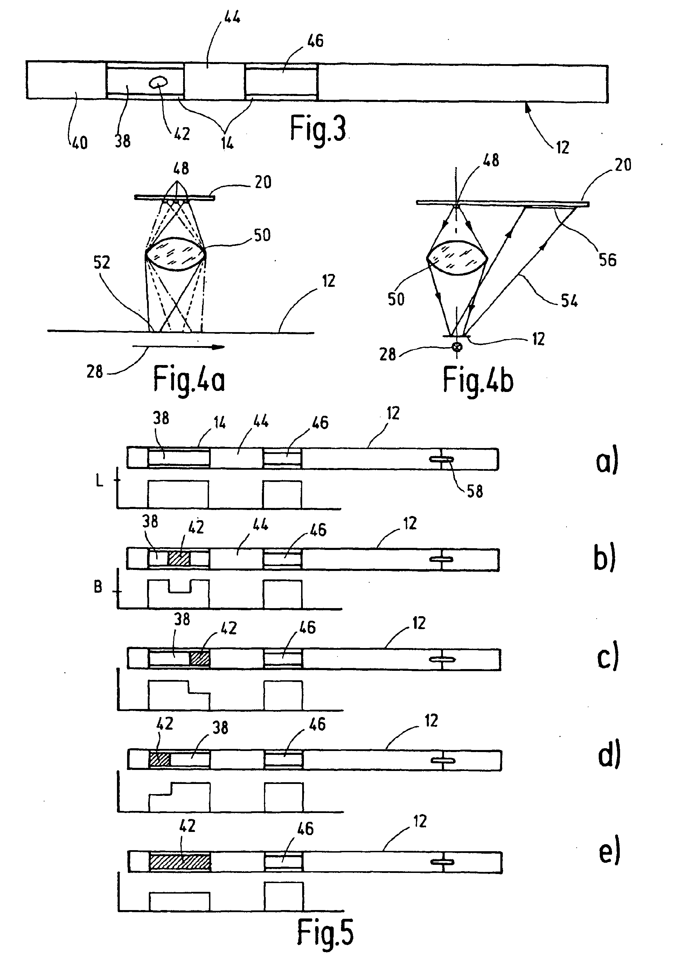

Wie am besten aus Fig. 3 ersichtlich, bestehen die Testelemente 14 als flächige Testfelder aus einer Reagenzschicht 38, die auf ein transparentes dünnes Trägerband 40 aufgebracht ist. Beim Auftragen eines Bluttropfens reagiert die Reagenzschicht 38 unter Farbumschlag auf einen in der Blutflüssigkeit enthaltenen Analyten (Glucose), so dass ein optischer Nachweis mittels der Messeinrichtung 18 möglich ist.

Für den Benutzer ist es wünschenswert, möglichst wenig Blut auftragen zu müssen, gleichzeitig aber eine große Auftragszone 38 zur Verfügung zu haben. Dadurch bedingt kann der mit Blut bzw. Probenflüssigkeit benetzte Bereich 42 von Messung zu Messung variieren, wobei durch die einen Mikroprozessor umfassende Positioniereinheit 20 sichergestellt wird, dass der Benetzungsbereich 42 an dem von der Aufgabestelle 34 entfernten Messort 36 zielgenau erfasst wird. Zu diesem Zweck wird der Bandantrieb 32 entsprechend angesteuert, so dass die Positionierung durch eine Transportunterbrechung erreicht wird, ohne dass zusätzliche mechanische Elemente notwendig wären. Zusätzlich zu der Auftragszone 38 weist das Testfeld 14 einen durch eine hydrophobe Flüssigkeitsbarriere 44 abgetrennten Referenzbereich 46 für eine Leerwertmessung auf.

Fig. 4 veranschaulicht die Feinpositionierung des Benetzungsbereichs 42 am Messort 36 mittels einer photometrisch arbeitenden Positioniereinrichtung 20. In der Seitenansicht nach Fig. 4a sind drei in Bandlaufrichtung 28 hintereinander angeordnete Leuchtdioden 48 gezeigt, die über eine Sammellinse 50 als entsprechende Leuchtflecken 52 hintereinander auf dem durchlaufenden Band 12 abgebildet werden. Der Abstand der äußeren LEDs 48 liegt dabei in der Größenordnung der gewöhnlichen Ausdehnung der Benetzungszone 42, d. h. im Bereich von wenigen Millimetern. Aus der Ansicht nach Fig. 4b in Bandlaufrichtung ist zu erkennen, dass das von den Leuchtdioden 48 eingestrahlte und von den Testelementen 14 auf dem Testband 12 diffus zurückgeworfene Licht 54 seitlich außerhalb der direkten Reflexion durch einen Photosensor 56 der Positioniereinrichtung 20 erfasst wird. Um mit nur einem Sensor 56 eine Zuordnung der erhaltenen Signale zu erlauben, können die LEDs 48 getrennt voneinander angesteuert werden. Grundsätzlich ist es möglich, dass der Sensor 56 neben der Benetzungserkennung zugleich der Nutzsignalerfassung dient. Um den unterschiedlichen Anforderungen gerecht zu werden, ist es jedoch zweckmäßig, hierfür einen gesonderten Detektor einzusetzen.

In Fig. 5 sind verschiedene Ausgangssignale des Sensors 56 in örtlicher Zuordnung zu den jeweils darüber gezeigten Benetzungszuständen des Testfelds 14 dargestellt. Bei vollständig trockenem Testfeld 14, d.h. Reaktionsbereich 38 und Referenzbereich 46 sind nicht mit Probenflüssigkeit beaufschlagt, wird in beiden Bereichen ein Leerwert L erfasst (Fig. 5a). Für den Fall der in Fig. 5b gezeigten mittigen Benetzung des Bereichs 38 wird dort ein geringerer Wert B der Remission entsprechend einem erfolgten Farbumschlag in der Reaktionsschicht gemessen. Fig. 5c und d zeigen die Situation einer Randbenetzung zu Beginn und am Ende des Reaktionsbereichs 38, während in Fig. 5e eine vollflächige Benetzung dargestellt ist.

Anzumerken ist noch, dass das Testband 12 eine dem Testelement 14 vorgeordnete Durchstechöffnung 58 für eine im Bereich der Probenaufgabestelle 34 angeordnete Stecheinheit aufweisen kann. Damit ist es mit einem einheitlichen Gerät möglich, mittels der Stecheinheit Kapillarblut beispielsweise aus einem Finger zu gewinnen, durch Bandvorlauf das Blut an der Probenaufgabestelle 34 mit einem Testelement 14 abzuholen und anschließend durch weiteren Bandvorlauf das betreffende Testelement am Messort 36 zu positionieren.

Hierbei kann die Feinpositionierung mittels der beschriebenen Reihe von Leuchtdioden 48 nach Fig. 4 erfolgen. Beim Durchlauf des Referenzbereichs 46 durch den ersten Leuchtfleck 52 (erzeugt von der in Fig. 4a rechten Leuchtdiode 48) wird der Leerwert L erfasst und für einen Vergleich abgespeichert. Läuft nun der Benetzungsbereich 42 ein, wird das aufgetragene Blut durch einen abgesenkten Plateau- bzw. Zielwert B, der kleiner L aber größer Null ist, erkannt. Wenn dieser Wert sich wieder ändert, unterbricht eine Steuereinheit der Positioniereinrichtung 20 den Bandantrieb 32, so dass der mit Blut benetzte Bereich 42 im Einstrahlbereich der mittleren LED 48 liegt, mit der dann auch die eigentliche Nachweismessung durchgeführt wird. Um die Zuverlässigkeit weiter zu erhöhen, kann eine dritte Leuchtdiode 48 vorgesehen sein (in Fig. 4a die linke LED), durch welche bei einem zusätzlichen Ansprechen auf Blut sicherstellt ist, dass der gesamte von der mittleren LED beleuchtete Nutzsignalerfassungsbereich in dem mit Blut benetzten Testfeldbereich 42 liegt.

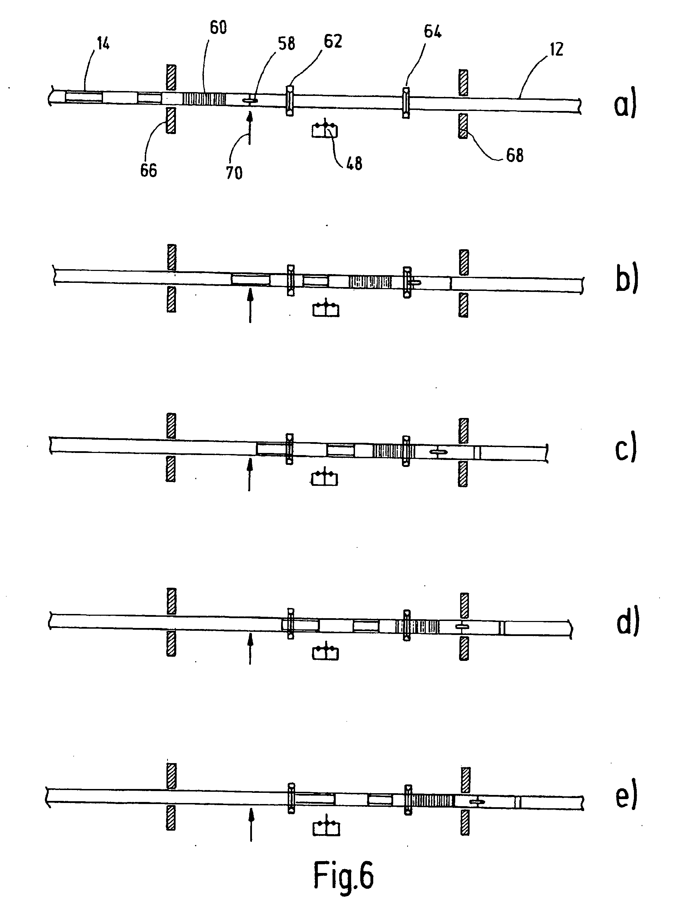

Bei dem in Fig. 6 gezeigten Ausführungsbeispiel sind gleiche Teile mit den gleichen Bezugszeichen wie vorstehend beschrieben versehen. Auf dem Testband 12 ist zusätzlich ein Strichmaßstab 60 in definiertem Abstand von einem jeweiligen Testelement 14 aufgebracht. Hier umfasst die Positioniereinheit 20 zwei Lichtschranken 62, 64 zum Abtasten des über eine Gehäusekante 66 bzw. Dichtung 68 durchlaufenden Testbandes 12. An der Probenaufgabestelle wird mittels der Stecheinheit 70 Blut gewonnen (Fig. 6a) und anschließend nach entsprechendem Bandvorlauf auf das Testfeld 14 aufgebracht (Fig. 6b). Im Zuge eines weiteren Bandvorlaufs wird mittels der ersten Lichtschranke 62 der Anfang des Benetzungsbereichs 42 erfasst (Fig. 6c). Zeitgleich wird an der zweiten Lichtschranke 64 über den Strichmaßstab 60 die zugehörige Bandposition ermittelt. Optional kann gemäß Fig. 6d auch die Endposition des Benetzungsbereichs bestimmt werden. In der Steuereinheit der Positioniereinrichtung 20 wird sodann anhand der ermittelten Bandpositionen der erforderliche Vorlauf für eine exakte Positionierung vor der Messeinrichtung 18 berechnet und durch entsprechendes weiteres Abtasten des Maßstabs 60 und Steuerung des Bandantriebs 32 ausgeführt, so dass in der angefahrenen Position gemäß Fig. 6e eine zielgenaue Punktmessung möglich ist.

Grundsätzlich können neben Blut auch andere Proben- bzw. Körperflüssigkeiten, insbesondere interstitielle Flüssigkeit in der beschriebenen Weise untersucht werden.

## Patentansprüche

1. System zur Untersuchung einer Probenflüssigkeit, insbesondere für Blutglucosebestimmungen, mit folgenden Merkmalen:
- einem eine Mehrzahl von Testelementen (14) aufweisenden, vorzugsweise in einer Bandkassette gelagerten Testband (12),
- einer die Testelemente (14) unter Bandvorlauf sukzessive an eine Probenaufgabestelle (34) transportierenden Bandtransportvorrichtung (16),
- einer die mit Probenflüssigkeit beaufschlagten Testelemente (14) an einem Messort (36) abtastenden Messeinrichtung (18), wobei der Messort (36) in Bandlaufrichtung im Abstand von der Probenaufgabestelle (34) angeordnet ist,
**gekennzeichnet durch**
- eine auf das Vorhandensein von Probenflüssigkeit auf dem Testelement (14) ansprechende Positioniereinrichtung (20), welche die Bandtransportvorrichtung (16) ansteuert, um den Bandtransport zur Positionierung eines Probenflüssigkeit tragenden Benetzungsbereichs (42) der Testelemente (14) am Messort (36) zu unterbrechen.

2. System nach Anspruch 1, **dadurch gekennzeichnet, dass** die Positioniereinrichtung (20) eine mit einem Bandantrieb (32) gekoppelte Steuereinheit für einen gezielten Bandstopp am Messort (36) aufweist.

3. System nach Anspruch 1 oder 2, **dadurch gekennzeichnet, dass** die Positioniereinrichtung (20) mindestens eine beim Durchlauf des Benetzungsbereichs (42) ansprechende optische Erfassungseinheit (48,56;62) aufweist.

4. System nach einem der Ansprüche 1 bis 3, **dadurch gekennzeichnet, dass** die Positioniereinrichtung (20) zwei auf Banddurchlaufstellen vor und nach einer Nutzsignalerfassungszone der Messeinrichtung (18) ausgerichtete Lichtquellen (48) aufweist.

5. System nach einem der Ansprüche 1 bis 4, **dadurch gekennzeichnet, dass** die Positioniereinrichtung (20) mehrere über eine gemeinsame Sammeloptik (50) in Bandlaufrichtung hintereinander auf dem Testband (12) als Leuchtflecken abgebildete Leuchtdioden (48) aufweist.

6. System nach einem der Ansprüche 1 bis 5, **dadurch gekennzeichnet, dass** die Positioniereinrichtung (20) mindestens einen Photosensor (56) zur vorzugsweise reflektometrischen Abtastung der Testelemente (14) aufweist.

7. System nach einem der Ansprüche 1 bis 6, **dadurch gekennzeichnet, dass** die Positioniereinrichtung (20) in einem unbenetzten Testfeldbereich (46) einen Leerwert (L) und im Benetzungsbereich einen davon verschiedenen Zielwert (B) erfasst.

8. System nach einem der Ansprüche 1 bis 7, **dadurch gekennzeichnet, dass** Messeinrichtung (18) einen von der Positioniereinrichtung (20) gesonderten Detektor für die Nutzsignalerfassung zum Nachweis eines Analyten in der Probenflüssigkeit besitzt.

9. System nach einem der Ansprüche 1 bis 8, **dadurch gekennzeichnet, dass** die Testelemente (14) durch vorzugsweise als Reagenzschicht ausgebildete flächige Testfelder (38) auf dem Testband (12) gebildet sind, und dass die Testfelder einen (38) vorzugsweise durch eine Flüssigkeitsbarriere (44) von der Probenflüssigkeit freigehaltenen Referenzbereich (46) für eine Leerwertmessung aufweisen.

10. System nach einem der Ansprüche 1 bis 9, **dadurch gekennzeichnet, dass** das Testband (12) mindestens abschnittsweise mit einer Bandmarkierung (60), insbesondere einem Strichmaßstab versehen ist, und dass die Positioniereinrichtung (20) ein Abtastmittel (62), insbesondere eine Lichtschranke zum Abtasten der Bandmarkierung (60) im Zuge des Bandvorlaufs aufweist.

11. System nach einem der Ansprüche 1 bis 10, **dadurch gekennzeichnet, dass** der Benetzungsbereich (42) der Testelemente (14) einen Durchmesser von weniger als 5 mm, insbesondere von 1 bis 2 mm aufweist.

12. System nach einem der Ansprüche 1 bis 11, **dadurch gekennzeichnet, dass** der Messort (36) in einem Abstand von 5 mm bis 5 cm von der Probenaufgabestelle (34) liegt.

13. System nach einem der Ansprüche 1 bis 12, **dadurch gekennzeichnet, dass** an der Probenaufgabestelle (34) eine Stecheinrichtung (70) zum Einstechen in ein Körperteil vorzugsweise durch das Testband (12) hindurch angeordnet ist.

14. Verfahren zur Untersuchung einer Probenflüssigkeit, insbesondere für Blutglucosebestimmungen, bei welchem eine Mehrzahl von Testelementen (14) auf einem Testband (12) unter Bandvorlauf sukzessive von einer Probenaufgabestelle (34) an einen im Abstand davon befindlichen Messort (36) transportiert werden, **dadurch gekennzeichnet, dass** durch eine auf das Vorhandensein von Probenflüssigkeit auf einem jeweiligen Testelement (14) ansprechende Positioniereinrichtung (20) der Bandtransport gesteuert wird, um einen Probenflüssigkeit tragenden Benetzungsbereich (42) des Testelements (14) am Messort (36) zu positionieren.

## Claims

1. System for analysing a sample liquid, in particular for blood glucose determinations, having the following features:
- a test tape (12) having a plurality of test elements (14) that is preferably stored in a tape cassette,
- a tape transport device (16) which successively transports the test elements (14) to a sample application site (34) while advancing the tape,
- a measuring device (18) which scans the test elements (14) loaded with sample liquid at a measuring site (36) wherein the measuring site (36) is located at a distance from the sample application site (34) in the direction of tape movement,
**characterized by**
- a positioning device (20) which responds to the presence of sample liquid on the test elements (14) and controls the tape transport device (16) in order to interrupt the tape transport to position a wetting area (42) of the test elements (14) carrying sample liquid at the measuring site (36).

2. System according to claim 1, **characterized in that** the positioning device (20) has a control unit coupled with a tape drive (32) for a targeted tape stop at the measuring site (36).

3. System according to claim 1 or 2, **characterized in that** the positioning device (20) has at least one optical detection unit (48, 56; 62) which responds when the wetting area (42) passes through.

4. System according to one of the claims 1 to 3, **characterized in that** the positioning device (20) has two light sources (48) aligned with tape passage positions before and after a useful signal detection zone of the measuring device (18).

5. System according to one of the claims 1 to 4, **characterized in that** the positioning device (20) has several light-emitting diodes (48) that are imaged consecutively on the test tape (12) in the direction of tape movement as light spots by common collection optics (50).

6. System according to one of the claims 1 to 5, **characterized in that** the positioning device (20) has at least one photosensor (56) for scanning the test elements (14) preferably by reflectometry.

7. System according to one of the claims 1 to 6, **characterized in that** the positioning device (20) detects a blank value (L) in an unwetted area of the test field (46) and a target value (B) that is different therefrom in the wetting area.

8. System according to one of the claims 1 to 7, **characterized in that** the measuring device (18) has a detector that is separate from the positioning device (20) for detecting the useful signals for the detection of an analyte in the sample liquid.

9. System according to one of the claims 1 to 8, **characterized in that** the test elements (14) are formed by flat test fields (38) that are preferably in the form of a reagent layer on the test tape (12) and that the test fields (38) have a reference area (46) for a blank value measurement that is preferably kept free from the sample liquid by a liquid barrier (44).

10. System according to one of the claims 1 to 9, **characterized in that** the test tape (12) is provided with a tape marking (60) in particular with a line scale at least on sections of the tape and that the positioning device (20) has a scanning means (62) and in particular a light barrier to scan the tape marking (60) while the tape is advanced.

11. System according to one of the claims 1 to 10, **characterized in that** the wetting area (42) of the test elements (14) has a diameter of less than 5 mm, in particular of 1 to 2 mm

12. System according to one of the claims 1 to 11, **characterized in that** the measuring site (36) is at a distance of 5 mm to 5 cm from the sample application site (34).

13. System according to one of the claims 1 to 12, **characterized in that** a lancing device (70) is arranged at the sample application site (34) for piercing a body part preferably through the test tape (12).

14. Method for analysing a sample liquid in particular for blood glucose determinations in which a plurality of test elements (14) on a test tape (12) are successively transported while advancing the tape from a sample application site (34) to a measuring site (36) that is located at a distance therefrom, **characterized in that** the tape transport is controlled by a positioning device (20) that responds to the presence of sample liquid on a respective test element (14) in order to position a sample liquid carrying wetting area (42) of the test element (14) at the measuring site (36).

## Revendications

1. Système pour l'analyse d'un échantillon liquide, en particulier pour la détermination de la glycémie, comportant les caractéristiques suivantes :
- une bande de test (12) présentant une pluralité d'éléments de test (14), insérée de préférence dans une cassette pour bande,
- un dispositif de transport de bande (16) transportant les éléments de test en faisant avancer la bande successivement vers un emplacement de chargement d'échantillon (34),
- un système de mesure (18) balayant les éléments de test (14) chargés en échantillon liquide au niveau d'une zone de mesure (36), la zone de mesure (36) étant disposée dans le sens d'avancée de la bande à distance de l'emplacement de chargement d'échantillon (34),
**caractérisé par**
- un système de positionnement (20) réagissant à la présence d'un échantillon liquide sur l'élément de test (14), lequel système de positionnement commande le dispositif de transport de bande (16) afin d'interrompre le transport de la bande au niveau de la zone de mesure (36) en vue du positionnement d'une zone d'humectage (42) des éléments de test (14) qui porte l'échantillon liquide.

2. Système selon la revendication 1, **caractérisé en ce que** le système de positionnement (20) présente une unité de commande couplée à un mécanisme d'entraînement de bande (32) pour un arrêt ciblé de la bande au niveau de la zone de mesure (36).

3. Système selon la revendication 1 ou 2, **caractérisé en ce que** le système de positionnement (20) présente au moins une unité de détection optique (48, 56 ; 62) réagissant lors de la traversée de la zone d'humectage (42).

4. Système selon l'une des revendications 1 à 3, **caractérisé en ce que** le système de positionnement (20) présente deux sources lumineuses (48) orientées vers les points de passage de la bande avant et après une zone de détection de signaux utiles du système de mesure (18).

5. Système selon l'une des revendications 1 à 4, **caractérisé en ce que** le système de positionnement (20) présente plusieurs diodes électroluminescentes (48) représentées l'une derrière l'autre sur la bande de test (12) par des points lumineux via une optique collectrice (50) dans le sens d'avancée de la bande.

6. Système selon l'une des revendications 1 à 5, **caractérisé en ce que** le système de positionnement (20) présente au moins un photocapteur (56) pour balayer de préférence par réflectométrie les éléments de test (14).

7. Système selon l'une des revendications 1 à 6, **caractérisé en ce que** le système de positionnement (20) détecte dans une zone de champ de test non humectée (46) une valeur à blanc (L) et dans la zone d'humectage une valeur cible (B) différente de celle-ci.

8. Système selon l'une des revendications 1 à 7, **caractérisé en ce que** le système de mesure (18) possède un détecteur distinct du système de positionnement (20) pour la détection de signaux utiles pour l'identification d'un analyte dans l'échantillon liquide.

9. Système selon l'une des revendications 1 à 8, **caractérisé en ce que** les éléments de test (14) sont formés par des champs de test plats (38) configurés de préférence en tant que couche réactive sur la bande de test (12), et **en ce que** les champs de test (38) présentent une zone de référence (46) de préférence maintenue exempte d'échantillon liquide par le biais d'une barrière contre le liquide (44) pour une mesure de valeur à blanc.

10. Système selon l'une des revendications 1 à 9, **caractérisé en ce que** la bande de test (12) est pourvue au moins partiellement d'un marquage de bande (60), en particulier d'un étalon gradué, et **en ce que** le système de positionnement (20) présente un moyen de balayage (62), en particulier une barrière lumineuse pour balayer le marquage de bande (60) dans le cadre de l'avancée de la bande.

11. Système selon l'une des revendications 1 à 10, **caractérisé en ce que** la zone d'humectage (42) des éléments de test (14) présente un diamètre inférieur à 5 mm, en particulier de 1 à 2 mm.

12. Système selon l'une des revendications 1 à 11, **caractérisé en ce que** la zone de mesure (36) se trouve à une distance de 5 mm à 5 cm de l'emplacement de chargement d'échantillon (34).

13. Système selon l'une des revendications 1 à 12, **caractérisé en ce qu'**un système de piqûre (70) est disposé au niveau de l'emplacement de chargement d'échantillon (34) pour piquer une partie du corps de préférence à travers la bande de test (12).

14. Procédé d'analyse d'un échantillon liquide, en particulier pour la détermination de la glycémie, dans lequel une pluralité d'éléments de test (14) sont transportés sur une bande de test (12) en faisant avancer la bande successivement depuis un emplacement de chargement d'échantillon (34) vers une zone de mesure (36) se trouvant à distance de celui-ci, **caractérisé en ce que** le transport de la bande est commandé par le biais d'un dispositif de positionnement (20) réagissant à la présence d'un échantillon liquide sur un élément de test (14) correspondant, afin de positionner au niveau de la zone de mesure (36) une zone d'humectage (42) de l'élément de test (14) portant l'échantillon liquide.
